Europäisches Patentamt

European Patent Office

Office européen des brevets

(19)

(11) Veröffentlichungsnummer : **0 374 379 B1**

# (12) EUROPÄISCHE PATENTSCHRIFT

(45) Veröffentlichungstag der Patentschrift :
**08.07.92 Patentblatt 92/28**

(51) Int. Cl.$^5$ : **C07C 37/74, C07C 39/02**

(21) Anmeldenummer : **89117354.4**

(22) Anmeldetag : **20.09.89**

(54) **Verfahren zum gleichzeitigen Entbasen und Entschwefeln von Phenolen.**

(30) Priorität : **19.12.88 DE 3842693**
**18.04.89 DE 3912670**

(43) Veröffentlichungstag der Anmeldung :
**27.06.90 Patentblatt 90/26**

(45) Bekanntmachung des Hinweises auf die
Patenterteilung :
**08.07.92 Patentblatt 92/28**

(84) Benannte Vertragsstaaten :
**AT BE CH DE ES FR GB GR IT LI LU NL SE**

(56) Entgegenhaltungen :
**DD-A- 212 506**
**DE-A- 2 758 566**

(73) Patentinhaber : **RÜTGERSWERKE**
**AKTIENGESELLSCHAFT**
**Mainzer Landstrasse 217**
**W-6000 Frankfurt am Main 1 (DE)**

(72) Erfinder : **Talbiersky, Jörg, Dr.**
**Reiherstrasse 93**
**W-4270 Dorsten (DE)**
Erfinder : **Wefringhaus, Bernhard**
**Westerfilder Strasse 62**
**W-4620 Castrop-Rauxel (DE)**
Erfinder : **Stolzenberg, Konrad, Dr.**
**Insterburger Weg 5**
**W-4355 Waltrop (DE)**
Erfinder : **Bergins, Wolfgang**
**Birkenstrasse 19**
**W-4620 Castrop-Rauxel (DE)**

EP 0 374 379 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Rohphenol, aber auch einzelne Phenolfraktionen, aus Teeren oder der hydrierenden Behandlung von Kohle enthalten als Verunreinigungen organische Basen und Schwefelverbindungen, die sich von den Phenolen nur sehr schwer und jeweils nur in einem Verfahrensschritt abtrennen lassen (vergl. Dierichs/Kubicka, Phenole und Basen, Akademie-Verlag Berlin, 1958, 344 ff).

Es ist daher Aufgabe der Erfindung ein Verfahren zur Behandlung von Phenolen bereitzustellen, bei dem mittels einer einfachen Behandlung der Gehalt an schwefel- und stickstoffhaltigen Verbindungen gleichzeitig wesentlich reduziert wird.

Die Lösung der Aufgabe erfolgt durch ein Verfahren gemäß der Ansprüche 1-7.

Es wurde gefunden, daß durch thermische Behandlung von Phenolen mit 1-5 Gew.-% Phthalsäureanhydrid (PSA) und anschließende fraktionierte Destillation sowohl der Basen- als auch der Schwefelgehalt der Phenole reduziert wird. Gleichzeitig wird zusätzlich die Farbstabilität erhöht. Eine weitere Verbesserung sowohl der Entbasungs- als auch der Entschwefelungswirkung sowie der Verbesserung der Farbstabilität wird erreicht, wenn man bei der thermischen Behandlung mit PSA noch geringe Mengen eines (0,01-0,5 Gew.-%, bezogen auf die Menge an Phenolen) Chinons zugibt. Dies ist insofern besonders überraschend, als durch alleinige Behandlung mit Chinonen kein Effekt erzielt wird.

Als Chinone sind alle para- oder amphi-Chinone einsetzbar wie z. B. Benzochinon, Naphthochinone, Anthrachinon oder entsprechende alkylsubstituierte Chinone. Die besten Ergebnisse werden bei Verwendung von Anthrachinon erhalten. Hier zeigte sich, daß als thermische Behandlung die bei einer Destillation unter milden Bedingungen erfolgende Wärmebehandlung ausreichend ist, wenn Rohphenole eingesetzt werden, die einen hohen Disulfidgehalt und einen relativ geringen Gehalt an Mercaptogruppen enthalten.

Die erfindungsgemäße Behandlung erfolgt dadurch, daß das Rohphenol oder die zu behandelnde Phenolfraktion mit dem Phthalsäureanhydrid bzw. mit Phthalsäureanhydrid und Chinon vermischt und unter Rühren mehrere (5-30) Stunden auf eine Temperatur im Bereich von 100-180 °C erhitzt wird. Danach wird das Reaktionsgemisch fraktioniert destilliert und die unerwünschten Verunreinigungen verbleiben im Destillationsrückstand.

In einer verbesserten Ausführungen werden die Phenole mit 1-5 Gew.-% Phthalsäureanhydrid bzw. mit 1-5 Gew.-% Phthalsäureanhydrid und 0,01-0,5 Gew.-% Chinon sowie ggf. einem aromatischen Kohlenwasserstoff versetzt und 5-30 h unter Rückfluß erhitzt. Danach wird fraktioniert, bevorzugt unter Vakuum, destilliert. Bevorzugte aromatische Kohlenwasserstoffe sind solche, die im Bereich von 100-150 °C sieden wie z. B. Toluol, Ethylbenzol oder Xylole.

In der bevorzugten Ausführung werden die Phenole, deren Gehalt an Disulfidgruppen durch vorhergehende oxidative Behandlung erhöht wurde, mit 1-5 Gew.-% Phthalsäureanhydrid und 0,01-0,5 Gew.-% Anthrachinon versetzt und bei möglichst niedriger Sumpftemperatur und kurzer Verweilzeit destilliert. Eine derartige Destillation unter milden Bedingungen ist z. B. eine Destillation unter Vakuum (30-80 mbar) bevorzugt in Kurzwegdestillationsapparaten wie Dünnschicht- oder Fallfilmverdampfer.

Eine weitere Vereinfachung in der Handhabung der hochschmelzenden Stoffe Phthalsäureanhydrid und Chinon besteht darin, diese Reagenzien in hochsiedenden Phenolen zu lösen. Dadurch sind die Reagenzien als Lösung einzusetzen, gut zu dosieren und es verbleibt ein gut handhabbarer Destillationsrückstand.

## Beispiele

Beispiel 1

Jeweils 4.000 g Rohphenol der folgenden Zusammensetzung

| Phenol | 61,6 % |
| o-Kresol | 10,35 % |
| m/p-Kresol | 20,15 % |
| 2,6-Dimethylphenol | 0,55 % |
| o-Ethylphenol | 0,20 % |
| 2,4/2,5-Dimethylphenol | 3,10 % |
| 3,5-Dimethylphenol + m/p-Ethylphenol | 2,45 % |
| 2,3-Dimethylphenol | 0,30 % |
| 3,4-Dimethylphenol | 0,30 % |
| p-Isopropylphenol | 0,90 % |
| $C_9$-Phenole | 0,10 % |

werden mit einer Lösung von 100 g Phthalsäureanhydrid (PSA), bzw. 100 g Phthalsäureanhydrid + 0,8 g Anthrachinon in hochsiedenden Phenolen sowie mit 200 ml Xylol (Isomerengemisch) versetzt und 24 h unter Rückfluß gekocht.

Anschließend wird das Reaktionsgemisch bei 133 mbar fraktioniert destilliert.

Die folgende Tabelle 1 zeigt die erhaltenen Ergebnisse im Vergleich zu einer Rohphenolprobe, die der gleichen Prozedur unterworfen wurde, der allerdings weder Phthalsäureanhydrid noch Anthrachinon zugesetzt worden war (Nullprobe).

Die entsprechenden Werte für die einzelnen Phenolfraktionen finden sich in den Tabellen 2 und 3.

Tabelle 4 zeigt die Farbstabilitäten der jeweiligen einzelnen Fraktionen gemessen nach 24 d nach der Methode ASTM 1686-61.

Tabelle 1

Kombinierte Entbasung und Entschwefelung von Rohphenol (Gesamtdestillat)

| Agens | PSA | PSA + Anthra- chinon | Null- probe |
|---|---|---|---|
| Destillationsausbeute | 95 % | 92 % | 93 % |
| Basenreduzierung im Gesamt- destillat bzg. auf Basenge- halt im Einsatz | 82 % | 86 % | 60 % |
| Basengehalt im Gesamt- destillat | 298 ppm | 240 ppm | 683 ppm |
| Schwefelreduzierung im Gesamtdestillat bzg. auf Schwefelgehalt im Einsatz | 52 % | 82 % | 31 % |
| Schwefelgehalt im Gesamt- destillat | 112 ppm | 43 ppm | 163 ppm |

Tabelle 2

Entbasung von Rohphenol

| Agens | PSA | PSA + Anthrachinon | Nullprobe |
|---|---|---|---|
| Basengehalt (ppm) im: | | | |
| Phenolvorlauf | 3 (-99)[1] | 6 (-97,9)[1] | 284 |
| Phenol | 6 - | 2 - | 6 |
| o-Kresol | 170 (-92,4) | 129 (-94,3) | 2251 |
| m/p-Kresol | 559 (-64,5) | 640 (-59,3) | 1574 |

[1]  Klammern: Abweichung des Basengehaltes von der Nullprobe in %

Tabelle 3

Entschwefelung von Rohphenol

| Agens | PSA | PSA + Anthra- chinon | Null- probe |
|---|---|---|---|
| Schwefelgehalt (ppm) im: | | | |
| Phenolvorlauf | 296 $(-82,6)^1$ | 327 $(-80,8)$[1] | 1708 |
| Phenol | 17 $(-37)$ | 16 $(-40,7)$ | 27 |
| o-Kresol | 16 $(-63,6)$ | 11 $(-75,0)$ | 44 |
| m/p-Kresol | 31 $(-44,8)$ | 17 $(-74,6)$ | 67 |

[1] Klammern: Abweichung des Schwefelgehaltes von der Nullprobe in %

Tabelle 4

Farbstabilität der Rohphenolfraktionen

| Agens | PSA | PSA +<br>Anthra-<br>chinon | Null-<br>probe |
|---|---|---|---|
| Farbe der Fraktion<br>nach 24 Tagen (Apha): | | | |
| Phenolvorlauf | 100 | 20 | > 250 |
| Phenol | 85 | 25 | > 250 |
| o-Kresol | 10 | 20 | 100 |
| m/p-Kresol | 7 | 25 | > 250 |

Beispiel 2

A    Eingesetztes Rohphenol (hohe Konversion in
      Disulfid)

      Gesamtschwefelgehalt:        165 ppm
      Mercaptoschwefel:              5 ppm
      Basen:                      1616 ppm

B    Eingesetztes Rohphenol (niedrige Konversion in
      Disulfid)

      Gesamtschwefel:              240 ppm
      Mercaptoschwefel:            184 ppm
      Basen:                      1010 ppm

In beiden Rohphenolen werden jeweils 2,5 Gew.-% Phthalsäureanhydrid und 0,2 Gew.-% Anthrachinon gelöst und diese Lösungen werden ohne weitere thermische Vorbehandlung innerhalb von 45 min bei 66 mbar

7

destilliert.

Die Destillatabnahme beträgt 90 %.

Die erhaltenen Ergebnisse sind in Tabelle 5 aufgeführt:

Tabelle 5

| Rohphenol-typ | Temperatur Kopf °C | Sumpf °C | Basen im Destillat ppm | Entbasungs-grad % | Schwefel im Destillat ppm | Entschwefelungs-grad % |
|---|---|---|---|---|---|---|
| A | 106-117 | 114-130 | 246 | 86,3 | 4 | 97,8 |
| B | 106-117 | 114-130 | 154 | 86,2 | 176 | 34,0 |

EP 0 374 379 B1

## Patentansprüche

1. Verfahren zum gleichzeitigen Entbasen und Entschwefeln von Phenolen, **dadurch gekennzeichnet**, daß die Phenole mit Phthalsäureanhydrid thermisch behandelt und destilliert werden.

2. Verfahren nach Anspruch 1, **dadurch gekennzeichnet**, daß die Phenole mit 1-5 Gew.-% Phthalsäureanhydrid und einem aromatischen Kohlenwasserstoff versetzt, 5-30 h unter Rückfluß erhitzt und anschließend unter Vakuum destilliert werden.

3. Verfahren nach den Ansprüchen 1 und 2, **dadurch gekennzeichnet**, daß die thermische Behandlung mit Phthalsäureanhydrid und einem Chinon erfolgt.

4. Verfahren nach Anspruch 3, **dadurch gekennzeichnet**, daß 0,01-0,5 Gew.-% Chinon, bezogen auf die Menge an Phenol, verwendet werden.

5. Verfahren nach den Ansprüchen 3 und 4, **dadurch gekennzeichnet**, daß als Chinon Anthrachinon verwendet wird.

6. Verfahren nach Anspruch 5, **dadurch gekennzeichnet**, daß die thermische Behandlung während einer Destillation unter milden Bedingungen erfolgt.

7. Verfahren nach den Ansprüchen 1 bis 6, **dadurch gekennzeichnet**, daß Phthalsäureanhydrid bzw. Phthalsäureanhydrid und Chinon in hochsiedenden Phenolen gelöst und als Lösung eingesetzt werden.

## Claims

1. A process for the simultaneous removal of bases and desulphurization of phenols, **characterized in that** the phenols are thermally treated with phthalic acid anhydride and are distilled.

2. A process according to Claim 1, **characterized in that** the phenols are reacted with from 1 to 5% by weight of phthalic acid anhydride and an aromatic hydrocarbon, are heated for 5 to 30 h under reflux and are then distilled under vacuum.

3. A process according to Claims 1 and 2, **characterized in that** the thermal treatment takes place with phthalic acid anhydride and a quinone.

4. A process according to Claim 3, **characterized in that** 0.01 to 0.5% by weight of quinone, relative to the quantity of phenol, is used.

5. A process according to Claims 3 and 4, **characterized in that** anthraquinone is used as quinone.

6. A process according to Claim 5, **characterized in that** the thermal treatment takes place during a distillation under mild conditions.

7. A process according to Claims 1 to 6, **characterized in that** phthalic acid anhydride or phthalic acid anhydride and quinone is or are dissolved in high-boiling phenols and used as a solution.

## Revendications

1. Procédé pour le débasage et la désulfuration simultanée de phénols, **caractérisé en ce que** les phénols sont traités thermiquement avec de l'anhydride de l'acide phtalique et distillés.

2. Procédé selon la revendication 1, **caractérisé en ce que** l'on ajoute aux phénols 1-5 % en poids de l'anhydride de l'acide phtalique et un hydrocarbure aromatique, en ce que l'on chauffe pendant 5-30 heures sous reflux et consécutivement en ce que l'on distille sous vide.

3. Procédé selon les revendications 1 et 2, **caractérisé en ce que** le traitement thermique s'effectue avec de l'anhydride de l'acide phtalique et une quinone.

4. Procédé selon la revendication 3, **caractérisé en ce que** l'on utilise 0,01-0,5 % en poids de quinone rapporté à la quantité de phénol.

5. Procédé selon les revendications 3 et 4, **caractérisé en ce qu'en** tant que quinone, on utilise de l'anthraquinone.

6. Procédé selon la revendication 5, **caractérisé en ce que** le traitement thermique s'effectue pendant une distillation modérée.

7. Procédé selon les revendications 1 à 6, **caractérisé en ce que** l'anhydride de l'acide phtalique ou l'anhydride de l'acide phtalique et la quinone sont dissous dans des phénols à point d'ébullition élevé et sont utilisés comme solution.